# EUROPEAN PATENT SPECIFICATION

(11) **EP 2 598 645 B1**
(45) Date of publication and mention of the grant of the patent: **21.05.2014**
(21) Application number: 11738435.4
(22) Date of filing: 26.07.2011
(51) Int. Cl.: C12P 19/04, C12P 19/18, C07H 15/10

(54) **Process for producing glycosides of acrylate derivates employing polysaccharides and glycosidases or glycosyltransferases**
Verfahren zur Herstellung von Glycosiden von Acrylderivaten unter Verwendung von Polysacchariden und Glycosidasen oder Glycosyltransferasen
Procédé de production de glycosides de dérivés d'acrylate en utilisant des polysaccharides et des glycosidases ou des glycosyltranférases

(30) Priority: 29.07.2010 EP 10171323
(43) Date of publication of application: 05.06.2013
(73) Proprietor: BASF SE, 67056 Ludwigshafen (DE)
(72) Inventor: KELLER, Harald, 67069 Ludwigshafen (DE); LOOS, Katja, NL-9711 Groningen (NL); KLOOSTERMAN, Wouter, 9714 JH Groningen (NL)
(86) International application number: PCT/EP2011/062780
(87) International publication number: WO 2012/013646

(56) References cited:
- EP-A1- 0 394 496
- US-A- 3 878 191
- DATABASE EPODOC EUROPEAN PATENT OFFICE; 7 January 1997 (1997-01-07), XP002659706, -& JP 9 003089 A (MITSUI NORIN KK; HAYASHIBARA BIOCHEM LAB) 7 January 1997 (1997-01-07)
- YORK, W.S. & HAWKINS, R.: "Preparation of oligomeric beta-glycosides from cellulose and hemicellulosic polysaccharides via the glycosyl transferase activity of a Trichoderma reesei cellulase", GLYCOBIOLOGY, vol. 10, no. 2, 2000, pages 193-201, XP008139551,

## Description

The invention relates to a method for producing an ethylenically unsaturated glycoside by reacting an ethylenically unsaturated compound with a polysaccharide in the presence of a glycosidase or a glycosyltransferase.

Polymers comprising sugar residues (saccharide copolymers) may share typical properties of saccharides such as good water solubility, high electrolyte stability, colloidal stability in hot water, strong interaction with surfaces such as cotton and non-toxicity. These specific properties open a variety of applications for such polymers. It is therefore of great interest to develop cost-effective methods for producing well-defined saccharide copolymers and their respective monomers. Such monomers may be polymerizable ethylenically unsaturated glycosides which result by glycosidic coupling a saccharide and an ethylenically unsaturated alcohol. The synthesis of such glycosides involves a number of challenges. There are many possibilities for the formation of positional isomers in which different hydroxyl groups of the saccharide become involved in bond formation. Further, there is the potential for the formation of different anomeric forms. Chemical synthesis of most monomers bearing sugar residues is therefore generally not feasible and results in poor yields of the desired monomer.

The application of enzymes has been considered an alternative approach for producing glycosidic monomers. In contrast to chemical synthesis, enzyme-catalyzed reactions of unprotected sugars usually yield a much more structurally homogeneous product due to their high stereoselectivity.

In general there are two approaches used for enzymatic synthesis of glycosides: thermodynamically controlled reverse hydrolysis and kinetically controlled transglycosylation. Approaches to use glycosidases, which catalyze glycoside hydrolysis *in vivo,* for glycoside synthesis by reverse hydrolysis are described, for example, by I. Gill and R. Valivety (Angew. Chem. Int. Ed. 39(21):3804-3808, 2000). Transfer of glycosyl units to non-sugar compounds with primary hydroxyl groups by enzymatically catalyzed transglycosylation has been shown, for example, by S. Matsumura et al. (Makromol. Chem., Rapid Commun. 14:55-58, 1993).

The document YORK, W.S. & HAWKINS, R.: "Preparation of oligomeric beta-glycosides from cellulose and hemicellulosic polysaccharides via the glycosyl transferase activity of a Trichoderma reesei cellulase" (GLYCOBIOLOGY, vol. 10, no. 2, 2000, pages 193-201) discloses a method for producing alkyl glycosides comprising reacting an acceptor alcohol with the polysaccharide xyloglucan in the presence of the glycosidase cellulase.

The patent document EP 0 394 496 A1 (31 October 1990; NIPPON FINE CHEMICAL CO., LTD.) discloses a method for producing the ethylenically unsaturated glycoside maltotriosyl ethoxyethyl methacrylate comprising chemically reacting the ethylenically unsaturated compound diethylene glycol methacrylate with methyl D-maltotrioside.

Polysaccharides such as cellulose or starch are inexpensive and readily available. It was therefore an object of the present invention to develop a method for the production of ethylenically unsaturated glycosides using polysaccharides as source of glycosyl units.

Thus, the present invention provides a method for producing an ethylenically unsaturated glycoside of formula I 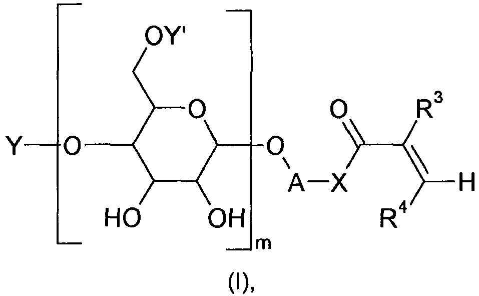 wherein
Y and Y' are independently H, or a monosaccharide or a linear or branched oligosaccharide comprising from 2 to 12 monosaccharide units, wherein at least one of Y and Y' is other than H;
m is an integer of from 0 to 3;
A is C₂₋₂₀ alkylene or -R⁶-O-[-R⁶-O-]ₓ-C₂₋₂₀ alkylene;
X is selected from the group consisting of -O-, -N H- and -NR⁵-,
R³ is selected from the group consisting of -H, and C₁₋₁₀ alkyl;
R⁴ is selected from the group consisting of -H, -COOH and -COO⁻ M⁺;
R⁵ is C₁₋₁₀ alkyl;
R⁶ is -C₂H₄- or -C₃H₆-;
M⁺ is selected from the group consisting of Li⁺, Na⁺, K⁺ and NH₄⁺; and
x is an integer of from 0 to 200;
comprising reacting an ethylenically unsaturated compound of formula II 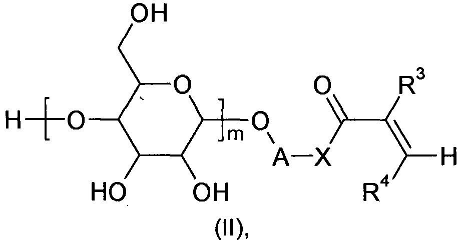 with a polysaccharide of comprising 10 or more monosaccharide units
in the presence of a glycosidase or a glycosyltransferase.

In preferred embodiments, Y' is H and Y is a monosaccharide, or a linear or branched oligosaccharide comprising from 2 to 12 monosaccharide units.

In preferred embodiments, Y is 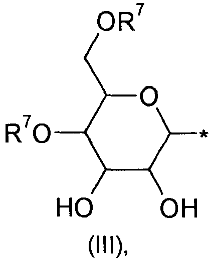 wherein R⁷ is -H or a radical of formula IV: 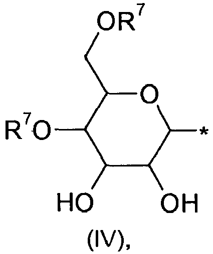 wherein in peripheral monosaccharide units R⁷ is H.

### Definitions

The term "monosaccharide" as used herein refers to a single unit of a polyhydroxyaldehyde forming an intramolecular hemiacetal the structure of which including a six-membered ring of five carbon atoms and one oxygen atom. Monosaccharides may be present in different diasteromeric forms, such as α or β anomers, and D or L isomers. An "oligosaccharide" consists of short chains of covalently linked monosaccharide units. Oligosaccharides comprise disaccharides which include two monosaccharide units as well as trisaccharides which include three monosaccharide units. A "polysaccharide" consists of long chains of covalently linked monosaccharide units.

The term "glycosidic bond" or "glycosidic linkage" is a type of chemical bond or linkage formed between the anomeric hydroxyl group of a saccharide or saccharide derivative (glycone) and the hydroxyl group of another saccharide or a non-saccharide organic compound (aglycone) such as an alcohol. The reducing end of the di- or polysaccharide lies towards the last anomeric carbon of the structure, and the terminal end is in the opposite direction.

An "enzymatically catalyzed" or "biocatalytic" method as used herein means that said method is performed under the catalytic action of an enzyme, in particular of a glycosidase or a glycosyltransferase. The method can be performed in the presence of said glycosidase or glycosyltransferase in isolated (purified, enriched) or crude form.

The term "glycosidase" also includes variants, mutants and enzymatically active portions of glycosidases.

Likewise, the term "glycosyltransferase" also includes variants, mutants and enzymatically active portions of glycosyltransferases.

Catalytic amounts of enzyme are expressed in "U" ("Unit" or "unit"), wherein 1 U equals the amount of enzyme which catalyses the reaction of 1 µmol substrate per minute under specific conditions (usually 37°C and pH 7.5). Thus, for example, 10 U glycosidase equals a catalytic amount of enzyme required for the reaction of 10 µmol saccharide substrate per minute. Catalytic amounts of maltogenic amylase can be expressed in "MANU" (Maltogenic Amylase Novo Unit), wherein 1 MANU equals the catalytic amount of enzyme required for the reaction of 1 µmol maltotriose per minute under standard conditions (10 mg/ml maltotriose, 37°C, pH 5.0, reaction time of 30 min). The catalytic amount of an enzyme can be determined by methods well know in the art.

The term "alkyl" comprises C₁₋₁₀ alkyl radicals which are linear or branched radicals having from 1 to 10 carbon atoms. Examples thereof are methyl, ethyl, n-propyl, isopropyl, n-butyl, 2-butyl, isobutyl or *tert*-butyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, nonyl, and decyl, as well as their constitutional isomers such as 2-ethylhexyl.

The term "alkylene" comprises C₂₋₂₀ alkylene diradicals which are linear or branched diradicals having from 1 to 20 carbon atoms.

The term "ethylenically unsaturated" refers to a compound comprising a non-aromatic C=C double bond. Specifically an "ethylenically unsaturated glycoside" as used herein refers to a glycoside consisting of a saccharide that is glycosidically linked to an ethylenically unsaturated alcohol.

In the method of the present invention, the polysaccharide acts as a source of saccharide units that are transferred to the ethylenically unsaturated compound of formula II to form the ethylenically unsaturated glycoside of formula I. The transferred saccharide units are monosaccharide units such as glucose, galactose, or mannose units; disaccharide units such as maltose, lactose or cellobiose units; trisaccharide units such as maltotriose units; or other oligosaccharide units such as maltohexaose, or a mixture thereof. For example, the transferred saccharide units are D-glucose, maltose, cellobiose, maltotriose, or maltohexaose units. The transferred oligosaccharide units can be linear or branched.

The polysaccharide used in the method of the present invention comprises 10 or more monosaccharide units. Typically, the number of monosaccharide units of the polysaccharide is from about 300 to about 200,000, for example from about 2,000 to about 200,000; from about 300 to about 3,000; or from about 300 to about 10,000. The size of polysaccharides, i.e. the number of monomer units, may be decreased by preprocessing, e.g. using acid and/or heat or enzymes such as α-amylases. The polysaccharides may be linear chains of monosaccharide units linked by (1→4)-glycosidic bonds; or may be branched by the formation of (1→6)-glycosidic bonds, e.g. every 24 to 30 monomer units. The terminal monosaccharide units of the saccharide chain (and side chains) are herein also called peripheral monosaccharide units.

In particular, the polysaccharide may be a compound of formula V 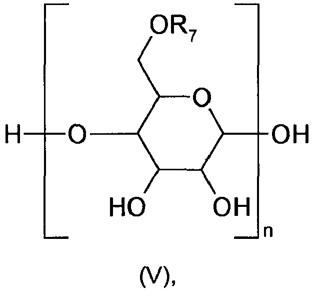 wherein R⁷ is -H or a radical of formula VI: 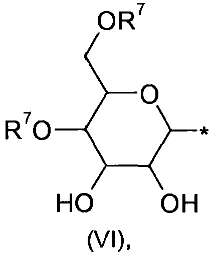 wherein in peripheral monosaccharide units R⁷ is H and n is such that the number of monosaccharide units encompassed by the recurring units in brackets is 10 or more, e.g., from about 300 to about 200,000.

Exemplary polysaccharides comprise starch, amylose, amylopectin, cellulose, and mixtures thereof.

In one embodiment, m is 0, i.e. the ethylenically unsaturated compound of formula II is an ethylenically unsaturated alcohol selected from hydroxyalkyl (meth)acrylates, N-hydroxyalkyl (meth)acrylamides, mono(hydroxyalkyl) esters of maleic acid, and salts thereof; or an ethoxylated, propoxylated or ethoxylated and propoxylated derivative thereof.

In other embodiments, m is 1, 2 or 3, *i.e.* the ethylenically unsaturated compound of formula II is an ethylenically unsaturated mono-, di- or triglycoside consisting of a mono-, di- or trisaccharide component and an alcohol component selected from the above ethylenically unsaturated alcohols.

Preferred ethylenically unsaturated alcohols of formula II are selected from 2-hydroxyethyl acrylate, 2-hydroxyethyl methacrylate, 3-hydroxypropyl acrylate, 3-hydroxypropyl methacrylate, N-(2-hydroxyethyl) acrylamide, N-(2-hydroxyethyl) methacrylamide, N-(3-hydroxypropyl) acrylamide, N-(3-hydroxypropyl methacrylamide, (2-hydroxyethyl) hydrogen maleate, and glycosides thereof.

In certain embodiments, A is C₂₋₆ alkylene, X is -O-, and R³ is -H or -CH₃.

In further embodiments, m is 1, Y' is H and Y is a monosaccharide or a linear or branched oligosaccharide comprising from 2 to 12 monosaccharide units, and preferably is a monosaccharide, a disaccharide or a trisaccharide.

In the method of the present invention, the reaction of polysaccharide and ethylenically unsaturated alcohol or glycoside is catalyzed by an enzyme selected from a glycosidase and a glycosyltransferase. Typically, enzymes show a high specificity regarding to the reactions they catalyze, the substrates that are involved in these reactions.

Glycosidases, a type of enzyme also known as glycoside hydrolases, are enzymes capable of catalyzing the hydrolysis of O- and S-glycosidic compounds. Further, glycosidases can be used for catalyzing the formation of glycosidic bonds through reverse hydrolysis, where the reaction equilibrium position is reversed, or transglycosylation, where a glycoside moiety is transferred from one glycoside, i.e. the donor glycoside, to another glycoside, i.e. the acceptor glycoside, to form a new glycoside. Glycosidases are assigned with enzyme classification number EC 3.2.1.x.

Glycosyltransferase are enzymes capable of catalyzing transglycosylation, a reaction wherein a glycoside moiety is transferred from one glycoside, *i.e.* the donor glycoside, to another glycoside, i.e. the acceptor glycoside, to form a new glycoside. Glycosyltransferases comprise hexosyltransferases which catalyze a transglycosylation wherein the transferred glycoside moiety is a hexose. Hexosyltransferases are assigned with enzyme classification number EC 2.4.1.x.

The enzyme may be used in a purified form, as an enriched concentrate or as a crude enzyme preparation.

Suitably, the glycosidase present in the method of the invention is selected from the group consisting of amylases, cellulases, glucosidases and galactosidases.

α-Amylase is an enzyme having enzyme classification number EC 3.2.1.1, and is also known as glycogenase, endoamylase, Taka-amylase A, or 1,4-α-D-glucan glucanohydrolase. α-Amylases are capable of catalyzing the endohydrolysis of (1→4)-α-D-glucosidic linkages in polysaccharides, containing three or more (1→4)-α-linked D-glucose units, such as starch and glycogen, thereby releasing reducing groups in the α-configuration.

β-Amylase is an enzyme assigned with enzyme classification number EC 3.2.1.2, and is also known as saccharogen amylase, glycogenase, or 1,4-α-D-glucan maltohydrolase. β-Amylases are capable of catalyzing the hydrolysis of (1→4)-α-D-glucosidic linkages in polysaccharides, such as starch and glycogen thereby releasing successive β-maltose units from the non-reducing ends of the polysaccharide chains.

Cellulase is an enzyme assigned with enzyme classification number EC 3.2.1.4, and is also known as endo-1,4-β-D-glucanase, β-1,4-glucanase, β-1,4-endoglucan hydrolase, celluase A, cellulosin AP, endoglucanase D, alkali cellulase, cellulase A 3, celludextrinase, 9.5 cellulase, avicelase, pancellase SS, or 1,4-(1,3;1,4)-β-D-glucan 4-glucanohydrolase. Cellulases are capable of catalyzing the endohydrolysis of (1→4)-β-D-glucosidic linkages in cellulose, lichenin and cereal β-D-glucans as well as 1,4-linkages in β-D-glucans also containing 1,3-linkages.

α-Glucosidase is an enzyme assigned with enzyme classification number EC 3.2.1.20, and is also known as maltase, glucoinvertase, glucosidosucrase, maltase-glucoamylase, α-glucopyranosidase, glucosidoinvertase, α-D-glucosidase, α-glucoside hydrolase, or α-1,4-glucosidase. α-Glucosidases are capable of catalyzing the hydrolysis of terminal, non-reducing (1→4)-linked α-D-glucose residues thereby releasing α-D-glucose.

β-Glucosidase is an enzyme assigned with enzyme classification number EC 3.2.1.21, and is also known as gentiobiase, cellobiase, emulsin, elaterase, aryl-β-glucosidase, β-D-glucosidase, β-glucoside glucohydrolase, arbutinase, amygdalinase, p-nitrophenyl β-glucosidase, primeverosidase, amygdalase, limarase, salicilinase, or β-1,6-glucosidase. β-Glucosidases are capable of catalyzing the hydrolysis of terminal, non-reducing β-D-glucosyl residues thereby releasing β-D-glucose.

α-Galactosidase is an enzyme assigned with enzyme classification number EC 3.2.1.22, and is also known as melibiase, α-D-galactosidase, α-galactosidase A, or α-galactoside galactohydrolase. α-Galactosidases are capable of catalyzing the hydrolysis of terminal, non-reducing α-D-galactose residues in α-D-galactosides, including galactose oligosaccharides, and galactomannans.

β-Galactosidase is an enzyme assigned with enzyme classification number EC 3.2.1.23, and is also known as lactase, β-lactosidase, maxilact, hydrolact, β-D-lactosidase, S 2107, lactozym, trilactase, β-D-galactanase, oryzatym, or sumiklat. β-Galactosidases are capable of catalyzing the hydrolysis of terminal non-reducing β-D-galactose residues in β-D-galactosides.

The glycosyltransferase present in the method of the invention may be a glycosyltransferase such as a hexosyltransferase, for example cyclomaltodextrin glucanotransferase or dextransucrase.

Cyclomaltodextrin glucanotransferase is an enzyme assigned with enzyme classification number EC 2.4.1.19, and is also known as *Bacillus macerans* amylase, cyclodextrin glucanotransferase, cyclodextrin glycosyltransferase, cyclomaltodextrin glucotransferase, cyclomaltodextrin glycosyltransferase, konchizaimu, cyclizing α-1,4-glucan 4-glycosyltransferase, BMA, CGTase, neutral-cyclodextrin glycosyltransferase, or 1,4-α-D-glucan 4-α-D-(1,4-α-D-glucano)-transferase. Cyclomaltodextrin glucanotransferases are capable of catalyzing the hydrolysis and the formation of (1→4)-α-D-glucosidic bonds, and in particular the formation of cyclic maltodextrins from polysaccharides as well as the disproportionation of linear oligosaccharides.

Dextransucrase is an enzyme assigned with enzyme classification number EC 2.4.1.5, and is also known as sucrose 6-glucosyltransferase, SGE, CEP, sucrose-1,6-α-glucan glucosyltransferase or sucrose:1,6-α-D-glucan 6-α-D-glucosyltransferase. Dextransucrases are capable of catalyzing the reaction: sucrose + [(1→6)-α-D-glucosyl]ₙ = D-fructose + [(1→6)-α-D-glucosyl]ₙ₊₁.

The enzyme may be dissolved in the reaction mixture or immobilized on a solid support which is contacted with the reaction mixture. If the enzyme is immobilised, it is attached to an inert carrier. Suitable carrier materials are known in the art. Examples for suitable carrier materials are clays, clay minerals such as kaolinite, diatomeceous earth, perlite, silica, alumina, sodium carbonate, calcium carbonate, cellulose powder, anion exchanger materials, synthetic polymers, such as polystyrene, acrylic resins, phenol formaldehyde resins, polyurethanes and polyolefins, such as polyethylene and polypropylene. For preparing carrier-bound enzymes the carrier materials usually are used in the form of fine powders, wherein porous forms are preferred. The particle size of the carrier material usually does not exceed 5 mm, in particular 2 mm. Further, suitable carrier materials are calcium alginate and carrageenan. Enzymes may directly be linked by glutaraldehyde. A wide range of immobilisation methods is known in the art (e.g. J. Lalonde and A. Margolin "Immobilization of Enzymes" in K. Drauz und H. Waldmann, Enzyme Catalysis in Organic Synthesis 2002, Vol.III, 991-1032, Wiley-VCH, Weinheim).

The enzymatically catalyzed reaction can be carried out batch wise, semi-batch wise or continuously. Reactants can be supplied at the start of reaction or can be supplied subsequently, either semi-continuously or continuously. The catalytic amount of glycosidase or glycosyltransferase required for the method of the invention depends on the reaction conditions, such as temperature, solvents and amount of substrate.

The reaction can be performed in aqueous media such as buffer. A buffer adjusts the pH of the reaction mixture to a value suitable for effective enzymatic catalysis. Typically the pH is in the range of about pH 4 to about pH 9, for example of about pH 5 to about pH 7. Suitable buffers comprise, but are not limited to, sodium acetate, tris(hydroxymethyl)aminomethane ("Tris") and phosphate buffers.

Optionally, the reaction takes place in the presence of a solvent mixture of water and a water miscible organic solvent at a weight ratio of water to organic solvent of from 0.1:1 to 9:1, for example from 1:1 to 3:1. The organic solvent is no primary or secondary alcohol and, accordingly, is non-reactive towards the polysaccharide. Suitable organic solvents comprise alkanones, alkylnitriles, tertiary alcohols and cyclic ethers, and mixtures thereof, for example acetone, acetonitrile, t-pentanol, t-butanol, 1,4-dioxane and tetrahydrofuran, and mixtures thereof. Generally, the use of organic solvents is not preferred.

A "water miscible organic solvent" is understood to mean an organic solvent that forms a homogeneous mixture with water at the weight ratio of water to organic solvent used.

The concentration and the ratio of the reactants, i.e. polysaccharide and ethylenically unsaturated compound of formula II, may be adapted to the optimum reaction conditions. For example, the initial polysaccharide concentration may be in the range of 10 to 400 g/I, relative to the total volume of the reaction mixture, for example 50 g/l.

The reaction temperature may be adapted to the optimum reaction conditions, which may depend on the specific enzyme applied. The reaction may expediently take place at temperatures between the freezing point of the reaction mixture and the denaturation temperature of the enzyme. Upon reaching the denaturation temperature the catalytic activity of the enzyme is lost. For example, the reaction may be performed at a temperature in the range from 0°C to 80°C, for example from 30°C to 65°C.
The process may proceed until equilibrium between reactants and products is achieved, but may be stopped earlier. Usual process times are in the range from 1 h to 96 h.

The methodology of the present invention can further comprise a step of recovering the produced ethylenically unsaturated glycoside of formula I. The term "recovering" includes extracting, harvesting, isolating or purifying the compound from the reaction mixture. Recovering the compound can be performed according to any conventional isolation or purification methodology known in the art including, but not limited to, treatment with a conventional resin (*e.g.*, anion or cation exchange resin, non-ionic adsorption resin, etc.), treatment with a conventional adsorbent (*e.g.*, activated charcoal, silicic acid, silica gel, cellulose, alumina, etc.), alteration of pH, solvent extraction (*e.g.*, with a conventional solvent such as an alcohol, ethyl acetate, hexane and the like), distillation, dialysis, filtration, concentration, crystallization, recrystallization, pH adjustment, lyophilization and the like.

Identity and purity of the isolated product may be determined by known techniques, like Thin Layer Chromatography (TLC), High Performance Liquid Chromatography (HPLC), gas chromatography (GC), Spectroscopy (*e.g*. IR, UV, NMR spectroscopy), coloring methods, N IRS, or enzymatic assays.

The examples described below are intended to illustrate the present invention.

### Example 1: Cellulase catalyzed synthesis of 2-(ß-glucosyloxy)-ethyl acrylate from cellulose

0.5 g cellulose was suspended in 10 ml 50 mM sodium acetate buffer at pH 5.0 containing 1 ml 2-hydroxyethyl acrylate. The reaction was started by addition of 0.010 g (60 U) cellulase from *Trichoderma reesei.* The reaction mixture was stirred for 3 days at 37°C. The product was detected by thin layer chromatography (TLC) (chloroform/methanol 4/1 (v/v), Rf 0.55). Unreacted cellulose was removed by filtration and the product was purified by column chromatography (silica gel, eluant: chloroform/methanol 7/1 (v/v)). Fractions containing the aimed product were pooled and the solvent was removed by rotary evaporation. Yield: 9.4 mg (2% of total cellulose).
¹H-NMR δ in ppm: 3.2-4.2 *Gluc*OC*H₂*CH₂R (8p); 4.38 GlucOCH₂C*H₂*R (2p Tri J 4.38 4.38 Hz); 4.50 Gluc*H*₁ (1 p Dou J 7.92 Hz); 5.99 *Hₜᵣₐₙₛ*CH=CHR (1p Dou J 10.42 Hz); 6.22 CH₂=C*H*R (1p DDou J 17.30 10.47 Hz); 6.46 *H_{cis}*CH=CHR (1p Dou J 17.31 Hz) ¹³C-NMR δ in ppm: 60.9 Gluc*C₆*; 64.4 OCH₂CH₂; 68.1 OCH₂CH₂; 69.8 Gluc*C₅*; 73.3 Gluc*C₂*; 75.9 Gluc*C₃*; 76.1 Gluc*C₄*; 102.7 GlucC*_{1ß}*; 127.6 H₂*C*=CHR; 132.9 H₂*C*=*C*HR; 168.6 O(O)CR
ESI-MS pos: calculated: 301.09 (C11H18O8Na); observed: 301.25

### Example 2: Amylase catalyzed synthesis of 2-(α-maltosyloxy)-ethyl acrylate from starch

5.0 g soluble starch was suspended in 50 ml 50 mM sodium acetate buffer at pH 5.0 containing 50 ml 2-hydroxyethyl acrylate. The reaction was started by addition of 10 ml (32000 MANU) maltogenic amylase from *Bacillus stearothermophilus.* The reaction mixture was stirred for 4 days at 55°C. The product was detected by TLC (chloroform/methanol 2/1 (v/v), Rf 0.45). The remaining 2-hydroxyethyl acrylate was removed by extraction with diethyl ether. The aqueous layer was concentrated by rotary evaporation at 30 °C. The unreacted starch was precipitated in isopropanol and removed by filtration. The filtrate was concentrated by rotary evaporation at 30 °C and the product was further purified by column chromatography (silica gel, eluant: chloroform/methanol 3/1 (v/v)). Fractions containing the aimed product were pooled and the solvent was removed by rotary evaporation.
Yield: 0.408 g (8%) Purity: >85 %
¹H-NMR δ in ppm: 3.2-4.2 *Gluc₁₋₂*O*CH₂*CH₂R (14p); 4.38-4.50 GlucOCH₂C*H₂*R (2p); 4.96 Gluc₁*H₁* (1p Dou J 3.75 Hz); 5.35 Gluc₂*H₁* (1p Dou J 3.76 Hz); 5.99 *Hₜᵣₐₙₛ*CH=CHR (1p Dou J 10.43 Hz); 6.21 CH₂=C*H*R (1p DDou J 17.29 10.45 Hz); 6.44 *H_{cis}*CH=CHR (1p Dou J 17.30 Hz)
¹³C-NMR δ in ppm: 60.6 Gluc₂*C₆*; 63.5 Gluc₁*C₆*; 64.1 OCH₂CH₂; 67.7 OCH₂CH₂; 69.5 Gluc₁*C₅*; 71.2 Gluc₂*C₅*; 72.0 Gluc₁*C₂*; 72.9 Gluc₁*C₃*; 73.1 Gluc₂*C₃*; 73.6 Gluc₂*C₂*; 76.3 Gluc₁*C₄*; 77.5 Gluc₂*C₄*; 98.2 Gluc₁C*_{1α}*; 100.1 Gluc₂C*_{1□}*; 127.7 H₂*C*=CHR; 132.8 H₂*C*=CHR; 168.6 O(O)CR
ESI-MS pos: calculated: 463.1422 (C17H28O13Na); observed: 463.3333

### Example 3: Cyclodextrin glycosyltransferase catalyzed synthesis of 2-(ß-maltriosyloxy)-ethyl acrylate from starch

0.634 g 2-(β-glucosyloxy)-ethyl acrylate and 1.0 g soluble starch were dissolved in 10 ml Tris-HCl buffer at pH 7.0 containing 0.01 M calcium chloride and 0.004 g MEHQ. The reaction was started by addition of 0.1 ml (60 U) cyclodextrin glycosyltransferase from *Bacillus macerans.* The reaction mixture was stirred for 1.5 h at 60°C. The product was detected by TLC (acetonitrile/water/ammonia 6/3/1 (v/v), Rf 0.27 and purified by column chromatography (silica gel, eluant: ethyl acetate/methanol 4/1 (v/v)). Fractions containing the aimed product were pooled and the solvent was removed by rotary evaporation. Product: α-D-glucopyranosyl-(1-4)-α-D-glucopyranosyl-(1-4)-ß-glucopyranosyl-oxyethyl acrylate. Yield: 0.241 g (38 w%) Purity: 95%
¹H-NMR δ in ppm: 3.2-4.2 *Gluc₁₋₃*OC*H₂*CH₂R (20p); 4.39 *Gluc*OC*H₂*C*H*₂R (2p Tri J 4.38 4.38 Hz); 4.50 Gluc₁*H₁*(1p Dou J 7.91 Hz); 5.39 Gluc₂₋₃*H₁*(2p Sin) 5.99 *Hₜᵣₐₙₛ*CH=CHR (1p Dou J 10.46 Hz); 6.22 CH₂=C*H*R (1p DDou J 17.29 10.46 Hz); 6.46 *H_{cis}*CH=CHR (1p Dou J 17.30 Hz)
¹³C-NMR δ in ppm: 60.7 Gluc₂₋₃*C₆*; 60.8 Gluc₁*C₆*; 64.4 O*C*H₂CH₂; 68.1 OCH₂*C*H₂; 69.5 Gluc₁*C₅*; 71.4 Gluc₂*C*₅; 71.7 Gluc₃*C₅*; 72.0 Gluc₁*C₂*; 72.9 Gluc₁*C₃*; 73.1 Gluc₂₋₃*C₃*; 73.5 Gluc₂*C₂*; 74.8 Gluc₃*C₂*; 76.3 Gluc₁*C₄*; 77.0 Gluc₂*C₄*; 77.1 Gluc₃*C₄*; 99.7 Gluc₃*C_{1α}*; 100.0 Gluc₂*C_{1α}*; 102.5 Gluc₁*C_{1β}*; 127.6 H₂*C*=CHR; 132.9 H₂*C*=*C*HR; 168.7 O(O)CR
ESI-MS pos: calculated: 625.1950 (C23H38O18Na); observed: 625.1957

## Claims

1. A method for producing an ethylenically unsaturated glycoside of formula I 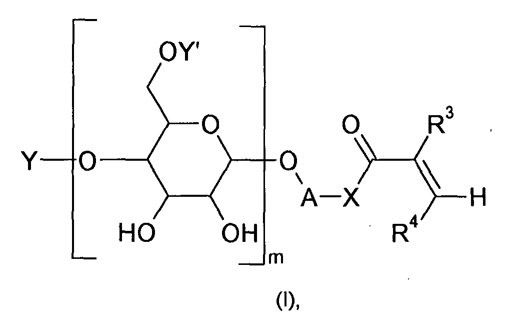 wherein
Y and Y' are independently H, or a monosaccharide or a linear or branched oligosaccharide comprising from 2 to 12 monosaccharide units, wherein at least one of Y and Y' is other than H;
m is an integer of from 0 to 3;
A is C₂₋₂₀ alkylene or -R⁶-O-[-R⁶-O-]ₓ-C₂₋₂₀ alkylene;
X is selected from the group consisting of -O-, -NH- and -NR⁵-,
R³ is selected from the group consisting of -H, and C₁₋₁₀ alkyl;
R⁴ is selected from the group consisting of -H, -COOH and -COO⁻ M⁺;
R⁵ is C₁₋₁₀ alkyl;
R⁶ is -C₂H₄- or -C₃H₆-;
M⁺ is selected from the group consisting of Li⁺, Na⁺, K⁺ and NH₄⁺; and
x is an integer of from 0 to 200;
comprising reacting an ethylenically unsaturated compound of formula II 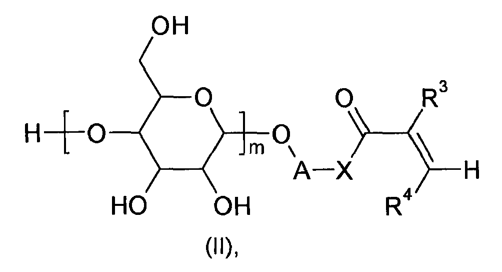 with a polysaccharide comprising 10 or more monosaccharide units in the presence of a glycosidase or a glycosyltransferase.

2. A method according to claim 1, wherein the polysaccharide is a compound of formula III: 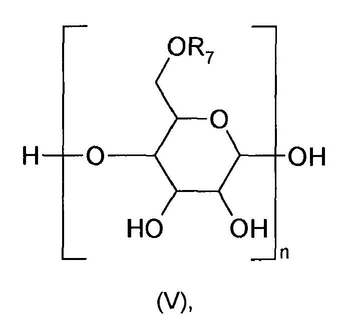 wherein R⁷ is -H or a radical of formula VI: 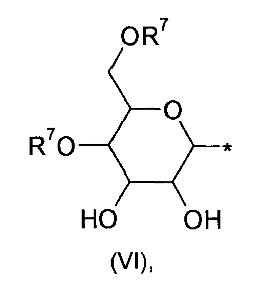 wherein in peripheral monosaccharide units R⁷ is H and n is such that the number of monosaccharide units encompassed by the recurring units in brackets is 10 or more.

3. The method of any one of claims 1 to 2, wherein Y' is H.

4. The method of any one of claims 1 to 3, wherein
A is C₂₋₆ alkylene;
X is -O-; and
R³ is -H, or -CH₃.

5. The method of any one of claims 1 to 4, wherein
m is 1; and
Y is selected from the group consisting of monosaccharides, disaccharides and trisaccharides.

6. The method of any one of claims 1 to 5, wherein the glycosidase is selected from the group consisting of amylases, cellulases, glucosidases and galactosidases.

7. The method of any one of claims 1 to 5, wherein the glycosyltransferase is selected from the group consisting of cyclomaltodextrin glucanotransferases.

8. The method of any one of claims 1 to 7, wherein the polysaccharide is selected from the group consisting of starch, amylose, amylopectin, cellulose, and mixtures thereof.

## Patentansprüche

1. Verfahren zur Herstellung eines ethylenisch ungesättigten Glycosids der Formel I 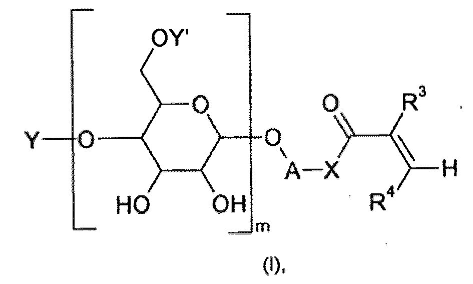 worin
Y und Y' unabhängig voneinander für H oder ein Monosaccharid oder ein lineares oder verzweigtes Oligosaccharid mit 2 bis 12 Monosaccharid-Einheiten stehen, wobei mindestens eine der Gruppen Y und Y' von H verschieden ist;
m für eine ganze Zahl von 0 bis 3 steht;
A für C₂₋₂₀-Alkylen oder -R⁶-O-[-R⁶-O-]ₓ-C₂₋₂₀-Alkylen steht;
X aus der Gruppe bestehend aus -O-, -NH- und -NR⁵-ausgewählt ist,
R³ aus der Gruppe bestehend aus -H und C₁₋₁₀-Alkyl ausgewählt ist;
R⁴ aus der Gruppe bestehend aus -H, -COOH und -COO⁻
M⁺ ausgewählt ist;
R⁵ für C₁₋₁₀-Alkyl steht;
R⁶ für -C₂H₄- oder -C₃H₆- steht;
M⁺ aus der Gruppe bestehend aus Li⁺, Na⁺, K⁺ und NH₄⁺ ausgewählt ist; und
x für eine ganze Zahl von 0 bis 200 steht;
bei dem man eine ethylenisch ungesättigte Verbindung der Formel II 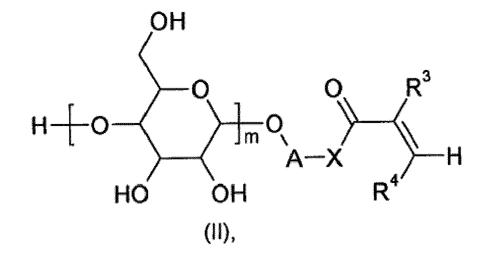 in Gegenwart einer Glycosidase oder einer Glycosyltransferase mit einem Polysaccharid mit 10 oder mehr Monosaccharid-Einheiten umsetzt.

2. Verfahren nach Anspruch 1, bei dem es sich bei dem Polysaccharid um eine Verbindung der Formel III: 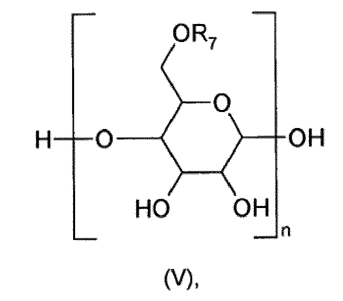 handelt, worin R⁷ für -H oder einen Rest der Formel VI steht: 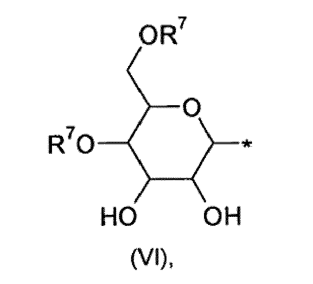 wobei in peripheren Monosaccharid-Einheiten R⁷ für H steht und n so beschaffen ist, dass die Zahl der durch die Wiederholungseinheiten in Klammern umfassten Monosaccharid-Einheiten 10 oder mehr beträgt.

3. Verfahren nach einem der Ansprüche 1 bis 2, bei dem Y' für H steht.

4. Verfahren nach einem der Ansprüche 1 bis 3, bei dem
A für C₂₋₆-Alkylen steht;
X für -0-steht; und
R³ für -H oder -CH₃ steht.

5. Verfahren nach einem der Ansprüche 1 bis 4, bei dem
M für 1 steht; und
Y aus der Gruppe bestehend aus Monosacchariden, Disacchariden und Trisacchariden ausgewählt ist.

6. Verfahren nach einem der Ansprüche 1 bis 5, bei dem man die Glycosidase aus der Gruppe bestehend aus Amylasen, Cellulasen, Glucosidasen und Galactosidasen auswählt.

7. Verfahren nach einem der Ansprüche 1 bis 5, bei dem man die Glycosyltransferase aus der Gruppe bestehend aus Cyclomaltodextringlucanotransferasen auswählt.

8. Verfahren nach einem der Ansprüche 1 bis 7, bei dem man das Polysaccharid aus der Gruppe bestehend aus Stärke, Amylose, Amylopektin, Cellulose und Mischungen davon auswählt.

## Revendications

1. Méthode de production d'un glycoside éthyléniquement insaturé de formule I 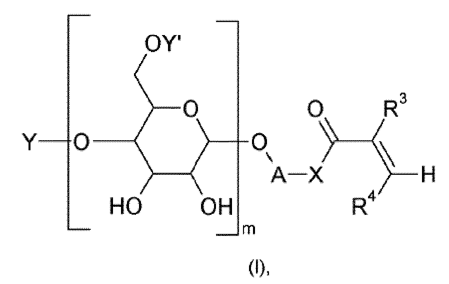 dans laquelle
Y et Y' sont indépendamment H, ou un monosaccharide ou un oligosaccharide linéaire ou ramifié comprenant de 2 à 12 unités de monosaccharide, où au moins un parmi Y et Y' est autre que H ;
m est un nombre entier allant de 0 à 3 ;
A est C₂₋₂₀ alkylène ou -R⁶-O-[-R⁶-O-]ₓ-C₂₋₂₀ alkylène ;
X est choisi dans le groupe constitué par -O-, -NH- et -NR⁵- ;
R³ est choisi dans le groupe constitué par -H et C₁₋₁₀ alkyle ;
R⁴ est choisi dans le groupe constitué par -H, -COOH et -COO⁻M⁺ ;
R⁵ est C₁₋₁₀ alkyle ;
R⁶ est -C₂H₄- ou -C₃H₆- ;
M⁺ est choisi dans le groupe constitué par Li⁺, Na⁺, K⁺ et NH₄⁺ ; et
x est un nombre entier allant de 0 à 200 ;
comprenant la réaction d'un composé éthyléniquement insaturé de formule II 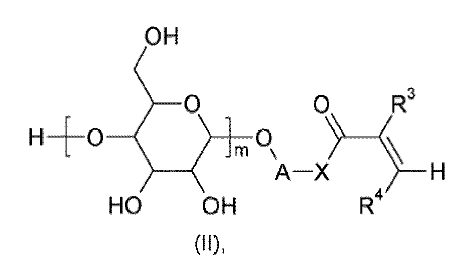 avec un polysaccharide comprenant 10, ou plus, unités de monosaccharide,
en présence d'une glycosidase ou d'une glycosyltransférase.

2. Méthode selon la revendication 1, dans laquelle le polysaccharide est un composé de formule III 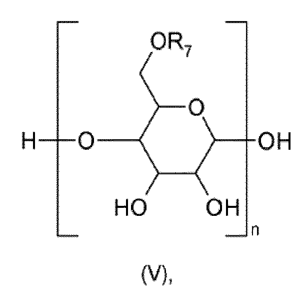 dans laquelle R⁷ est -H ou un radical de formule VI 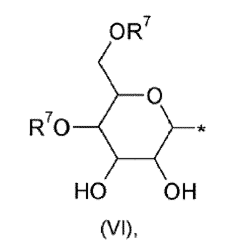 dans laquelle, dans les unités de monosaccharide périphériques, R⁷ est H et n est tel que le nombre d'unités de monosaccharide englobées par les unités récurrentes entre crochets est de 10, ou plus.

3. Méthode selon l'une quelconque des revendications 1 à 2, dans laquelle Y' est H.

4. Méthode selon l'une quelconque des revendications 1 à 3, dans laquelle
A est C₂₋₆ alkylène ;
X est -0- ; et
R³ est -H ou -CH₃.

5. Méthode selon l'une quelconque des revendications 1 à 4, dans laquelle
m vaut 1 ; et
Y est choisi dans le groupe constitué par les monosaccharides, les disaccharides et les trisaccharides.

6. Méthode selon l'une quelconque des revendications 1 à 5, dans laquelle la glycosidase est choisie dans le groupe constitué par les amylases, les cellulases, les glucosidases et les galactosidases.

7. Méthode selon l'une quelconque des revendications 1 à 5, dans laquelle la glycosyltransférase est choisie dans le groupe constitué par les cyclomaltodextrine glucanotransférases.

8. Méthode selon l'une quelconque des revendications 1 à 7, dans laquelle le polysaccharide est choisi dans le groupe constitué par l'amidon, l'amylose, l'amylopectine, la cellulose, et des mélanges de ceux-ci.
